# EUROPEAN PATENT APPLICATION

(11) **EP 4 316 471 A1**
(43) Date of publication of application: **07.02.2024**
(21) Application number: 22781600.6
(22) Date of filing: 30.03.2022
(51) Int. Cl.: A61K 9/51, A61K 9/00, A61K 31/167, A61K 31/513, A61K 31/155, A61K 31/675, A61P 1/00, A61P 43/00

(54) **ORAL NANOPARTICLES FOR BIOACTIVE COMPOUND, AND METHOD OF PREPARING SAME**

(30) Priority: 01.04.2021 KR 20210042768; 29.03.2022 KR 20220038802
(71) Applicant: SNJ Pharma Inc, Torrance, CA 90502 (US)
(72) Inventor: HONG, Seong Tshool, Jeonju-si Jeollabuk-do 54942 (KR); KIM, Hyeon Jin, Torrance, California 90502 (US); HONG, Jinny, Corona, California 92882 (US); ZHAI, Chongkai, Jeonju-si Jeollabuk-do 54907 (KR); WANG, Mingda, Jeonju-si Jeollabuk-do 54907 (KR)
(74) Representative: Ter Meer Steinmeister & Partner
(86) International application number: PCT/KR2022/004490
(87) International publication number: WO 2022/211481

(57) **Abstract**

The present invention relates to: oral nanoparticles for enabling oral administration of a bioactive compound which is typically administered by injection due to having low bioavailability attributable to issues with solubility, disintegration in the digestive tract, and intestinal permeability; a method of formulating the oral nanoparticles; and a use of the oral nanoparticles. In addition, the present invention relates to: oral nanoparticles capable of maintaining or protecting the balance in the gut microbiome by minimizing the exposure of the gut microbiome to a bioactive compound; a formulation; and a use of same.

## Description

### Technical Field

The present disclosure relates to an oral nanoparticle for improving the bioavailability of a bioactive compound, which is low due to issues in solubility, degradation in the digestive tract, and intestinal permeability, and to a method of preparing the same. In addition, the present disclosure relates to an oral nanoparticle capable of maintaining or protecting the balance in the gut microbiome by minimizing exposure of a bioactive compound to the gut microbiome, and to a method of preparing the same.

### Background Art

Drugs, nutritional supplements, and dietary supplements containing bioactive compounds for human health are most preferred oral medications in tablet form. The manufacturing costs of injections are typically 10 times higher than those of tablets, so injections are also highly priced. In addition, unlike oral medications that are easy to take, injections are necessary to be administered by visiting a hospital in person, so additional time and costs are required for patients. Furthermore, pain is caused inevitably in the process of administration. As described above, oral medications have excellent advantages compared to injections. Therefore, bioactive compounds required to be absorbed into the body are most commonly formulated into tablet forms first.

However, a considerable number of bioactive compounds in medications, nutritional supplements, and dietary supplements have extremely low oral bioavailability. As a result, such bioactive compounds are difficult to be formulated into oral medications and thus are developed only in injection form. Drugs having low oral bioavailability are characterized by being degradable by digestive enzymes, having low permeability to the blood in the intestine, or having low solubility due to the characteristics of the drugs. Thus, in the case of oral administration rather than the case of direct injection into the blood, the amount absorbed into the blood is much less than the dosage. Accordingly, the situation is that drugs having low bioavailability are being developed only as injections or oral medications having low bioavailability.

Another problem with drugs having low bioavailability is that only a portion of intake is absorbed, and the remainder is continuously exposed to the digestive tract, leading to problems in gut health, such as indigestion and the like. In addition, with intestinal microorganisms residing in the digestive tract being exposed to drugs present in the digestive tract, the survival rate of each species is greatly affected, which causes gut microbiome imbalance and thus leads to all types of health issues.

The gut microbiome has a profound impact not only on all kinds of diseases, such as diabetes, dementia, cancer, hypertension, hyperlipidemia, and the like, but also on the overall health of humans, including digestion. Due to such characteristics of the gut microbiome, maintaining a healthy gut microbiome is essential for health. Chemical compounds ingested by humans are absorbed into the body through the process of being evenly mixed with human intestinal contents. Therefore, the ingested chemical compounds severely affect the survival, colonization, and the like of intestinal microorganisms, causing the gut microbiome to be out of equilibrium. In particular, when taking an antibiotic with a microbicidal effect as an oral tablet, certain species of intestinal microorganisms are killed, causing the gut microbiome to be out of balance. As a result, not only digestive tract-related diseases, such as diarrhea, abdominal pain, and the like, but also various health issues related to the gut microbiome occur. Therefore, there is an urgent need to develop a technology for improving the bioavailability of a bioactive compound by applying the bioactive compound to the gut microbiome such that the gut microbiome is kept from being affected.

Due to the reasons described above, efforts have been made to develop oral preparations having excellent bioavailability, and the following technologies have been developed.

U.S. Patent No. 7060708 is an invention of allowing an amino acid to be covalently bonded (conjugated) to a compound to be absorbed into the body so that the bonded compound is also absorbed into the body when the amino acid is absorbed in the digestive tract. In this invention, when chemical changes occur due to the covalent bond, the unique properties of the compound are changed, resulting in problems of reduced activity or modification and side effects.

All U.S. Patent Nos. 7144877, 7598235, 7678782, and the like propose novel compound structures in which drug-linker-bile acid-based compounds are chemically linked to each other by covalent bonds. In this invention, there is a problem in that the drug is degraded by digestive enzymes in the digestive tract. In addition, when chemical changes occur due to the covalent bond, the unique properties of the compounds are changed, resulting in problems of reduced activity or modification and side effects.

U.S. Patent No. 7736679 proposes a method of improving bioavailability by increasing the absorption rate of curcumin when preparing a mixture by adding turmeric oil to curcumin. This invention is limited to the case involving curcumin and turmeric oil, and the degree of improving the bioavailability is insignificant.

U.S. Patent Publication No. 20190247313 provides a nanoparticle prepared by applying electrical stimulation to a drug present in a core portion to show a positive charge and then mixing the drug with a negatively charged polymer compound to which bile acid is covalently bonded. This invention has limitations in delivering the drug to the human body due to the excessively small amount of the drug capable of being encapsulated. In addition, the product generated by covalently bonding the negatively charged polymer and the bile acid is a novel chemical compound, so unexpected side effects may occur.

U.S. Patent Publication No. 20200009067 provides a drug solution prepared by dissolving a hydrophobic drug in a mixed solution consisting of three components, a lipid, a surfactant, and an emulsification solvent. During oral ingestion, the drug solution of this invention is mixed with digestive juices in the body and increases the absorption, so the bioavailability may be improved. However, this invention is limited to the case involving hydrophobic drugs. In addition, there is a limitation that the improvement in bioavailability is insignificant.

As described above, in technologies for improving bioavailability, a method of covalently bonding a compound used to improve solubility, intestinal permeability, absorption rate, and the like is being used. However, during chemical bonding (covalent bonding), the compound is transformed into a new chemical entity, causing changes in the structure and chemical properties of the original compound. Thus, there is a problem in that the unique properties of the drug may be lost, or unexpected toxicity and immune-related side effects may occur. In other words, the existing technologies not only fail to improve the bioavailability of oral medications but also fail to solve the problem in the gut microbiome resulting from oral administration.

In order to replace injectable drugs with oral tablets, problems in existing oral administration need to be fundamentally solved. In other words, there is an urgent need for a technology that dramatically improves the bioavailability for use after being absorbed into the body without causing any modification of orally administered compounds. In addition, a technology for maintaining the balance in the gut microbiome by minimizing exposure of orally administered compounds to intestinal microorganisms is important. There is an urgent need to develop such technologies for improving the health of mankind.

### Disclosure

### Technical Problem

The inventors of the present disclosure made intensive efforts to solve the problem of low bioavailability of bioactive compounds and the problem of causing the gut microbiome to be out of equilibrium. Therefore, an objective of the present disclosure is to provide an oral nanoparticle in which the bioavailability of a bioactive compound is improved and a method of preparing the same. In addition, another objective of the present disclosure is to provide an oral nanoparticle capable of maintaining and protecting the balance in the gut microbiome by minimizing exposure of an orally administered compound to intestinal microorganisms and a method of preparing the same.

### Technical Solution

In order to accomplish the objectives described above, the present disclosure provides an oral nanoparticle, which enables the bioavailability of a bioactive compound to be dramatically improved and the balance in the gut microbiome to be maintained and protected, and a method of preparing the same.

The inventors of the present disclosure paid attention to the fact that bile acid is amphiphilic, characterized by being composed of a hydrophobic portion and a hydrophilic portion. Hence, the inventors of the present disclosure hypothesized that the balance in the gut microbiome is maintained because once a nanoparticle having a structure in which the hydrophilic portion of bile acid is present on the periphery of the nanoparticle while the hydrophobic portion on the inside surrounds the compound is prepared, even when reaching a digestive organ by oral administration, the compound encapsulated inside the nanoparticle is kept from being degraded by digestive juices of digestive organs and being exposed to the gut microbiome.

In addition, the inventors of the present disclosure paid attention to the fact that more than 90% of bile acids secreted from the digestive tract are reabsorbed into the blood through an active uptake/transport system in the enterohepatic circulation. Bile acid is one type of emulsifier that allows the nutrients of lipid components to be well-mixed with digestive enzymes. Accordingly, the inventors of the present disclosure hypothesized that when nanoformulation is enabled by applying only bile acid to a bioactive compound without causing any modification, both of the bioactive compound and the bile acid are allowed to be reabsorbed from the digestive tract into the blood.

Hence, the inventors of the present disclosure conducted several experiments and succeeded in oral nanoparticle formulation using bile acid for encapsulation. In addition, it was confirmed through experiments of the prepared oral nanoparticle that a compound encapsulated with the bile acid was almost completely absorbed into the body.

In order to accomplish the objectives described above, the nanoparticle of the present disclosure is formed by non-covalently bonding ① a bioactive compound intended to be absorbed into the body, and ② bile acid surrounding and encapsulating the same. The oral nanoparticle of the present disclosure is characterized by 1) dramatically improving the bioavailability of the encapsulated compound, 2) preventing modification or degradation in digestive organs by minimizing the exposure of the encapsulated compound to digestive juices, 3) increasing intestinal permeability by reabsorbing both of the encapsulated compound and the bile acid into the blood, and 4) maintaining and protecting the balance in the gut microbiome by minimizing the exposure to intestinal microorganisms with the improved bioavailability of the encapsulated compound.

In particular, unlike in existing technologies, the compound and the bile acid constituting the nanoparticle of the present disclosure are only non-covalently bonded without involving any covalent bond. As a result, the present disclosure is characterized in that 1) the efficacy of the chemical compound is maintained without causing any chemical modification, and 2) new chemical entities are not produced, thus having no unpredictable side effects.

Therefore, the present disclosure provides an oral nanoparticle with innovatively improved bioavailability and a method of preparing the same.

### Advantageous Effects

An oral nanoparticle, according to the present disclosure, has the following effects of 1) dramatically improving the bioavailability of an encapsulated compound, 2) preventing modification or degradation in digestive organs by minimizing the exposure of the encapsulated compound to digestive juices, 3) increasing the intestinal permeability by reabsorbing both of the encapsulated compound and bile acid into the blood, and 4) maintaining and protecting the balance in the gut microbiome by minimizing the exposure of the encapsulated compound to intestinal microorganisms.

### Description of Drawings

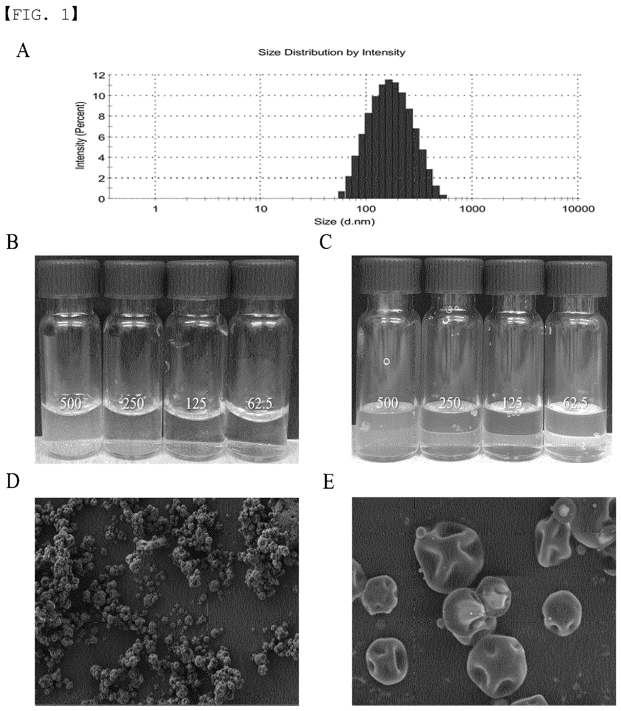

FIG. 1 shows confirmation results of comparison with original niclosamide nanoparticles not involving nanoparticle formation after preparing an aqueous solution (labeled concentration mg/ml) with niclosamide nanoparticles prepared according to Example 1 of the present disclosure (FIG. 1A: particle size analysis of prepared niclosamide nanoparticles, FIG. 1B: niclosamide solution with identified precipitates, FIG. 1C: niclosamide nanoparticle solution free of precipitates, FIG. 1D: low-magnification electron microscope image of niclosamide nanoparticles, and FIG. 1E: high-magnification electron microscope image of niclosamide nanoparticles);

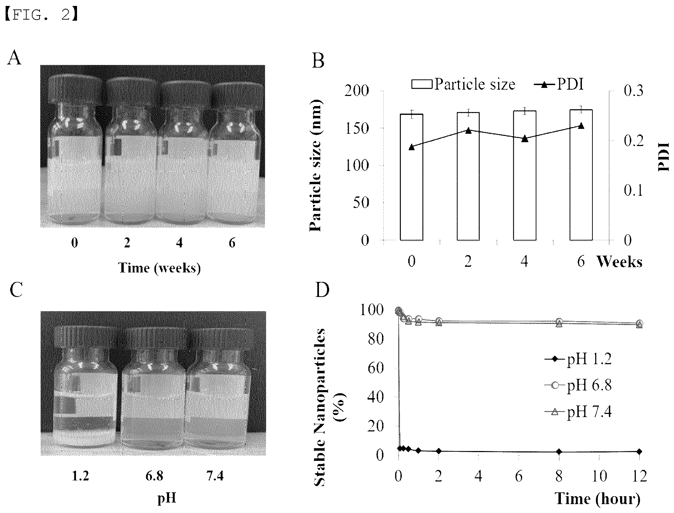

FIG. 2 shows confirmation results of the structural stability of niclosamide nanoparticles prepared according to Example 1 of the present disclosure when stored for a long term or at various pH conditions (FIG. 2A: niclosamide nanoparticle solution stored for long term, FIG. 2B: particle size analysis results of niclosamide nanoparticle solution stored for long term, FIG. 2C: niclosamide nanoparticle solution at various pH conditions, and FIG. 2D: particle size analysis results of niclosamide nanoparticle solution at various pH conditions); and

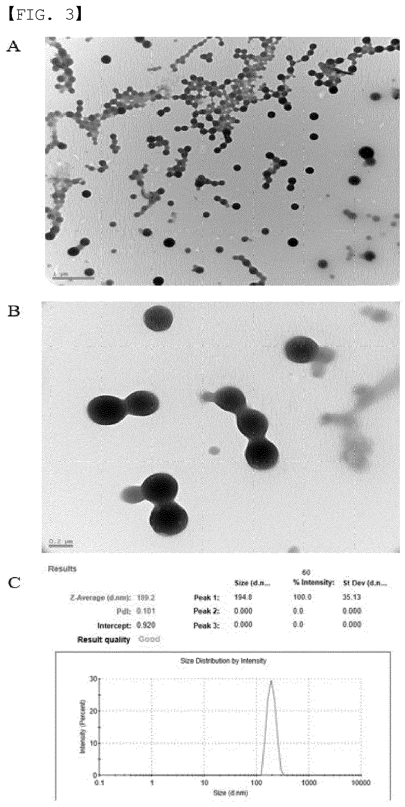

FIG. 3 shows electron microscope images of paclitaxel nanoparticles prepared according to Example 1 of the present disclosure and the analysis results thereof using a particle size analyzer (FIG. 3A: low-magnification electron microscope image of paclitaxel nanoparticles, FIG. 3B: high-magnification electron microscope image of paclitaxel nanoparticles, and FIG. 3C: analysis results of paclitaxel nanoparticles prepared with an average particle size of 189 nm);

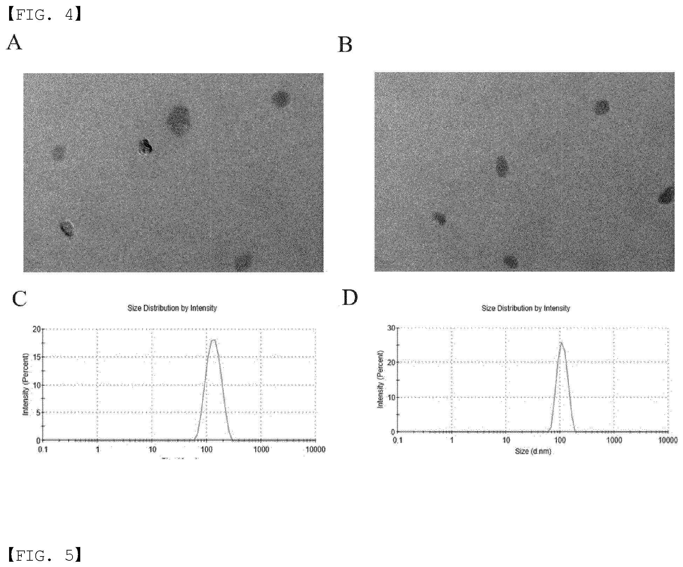

FIG. 4 shows electron microscope images of 5-fluorouracil nanoparticles and metformin nanoparticles prepared according to Example 2 of the present disclosure and the analysis results thereof using a particle size analyzer (FIG. 4A: electron microscope image of 5-fluorouracil nanoparticle, FIG. 4B: electron microscope image of metformin nanoparticles, FIG. 4C: analysis results of 5-fluorouracil nanoparticles prepared with an average particle size of 119 nm, and FIG. 4D: analysis results of metformin nanoparticles prepared with an average particle size of 108 nm);

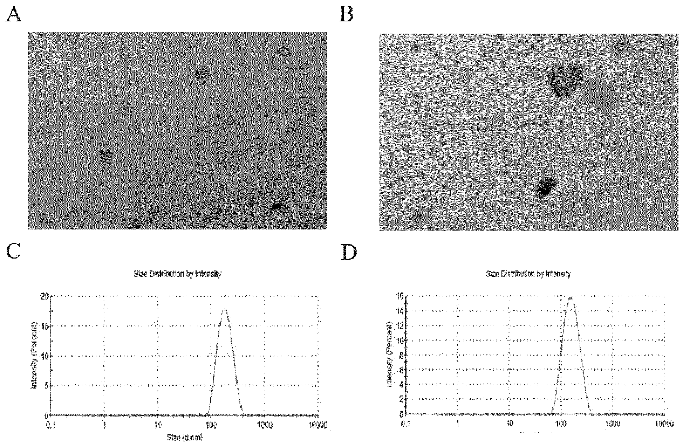

FIG. 5 shows electron microscope images of azithromycin nanoparticles and ciprofloxacin nanoparticles prepared according to Example 3 of the present disclosure and the analysis results thereof using a particle size analyzer (FIG. 5A: electron microscope image of azithromycin nanoparticles, FIG. 5B: electron microscope image of ciprofloxacin nanoparticles, FIG. 5C: nanoparticle analysis results of azithromycin nanoparticles prepared with an average particle size of 172 nm, and FIG. 5D: analysis results of ciprofloxacin nanoparticles prepared with an average particle size of 142 nm) ;

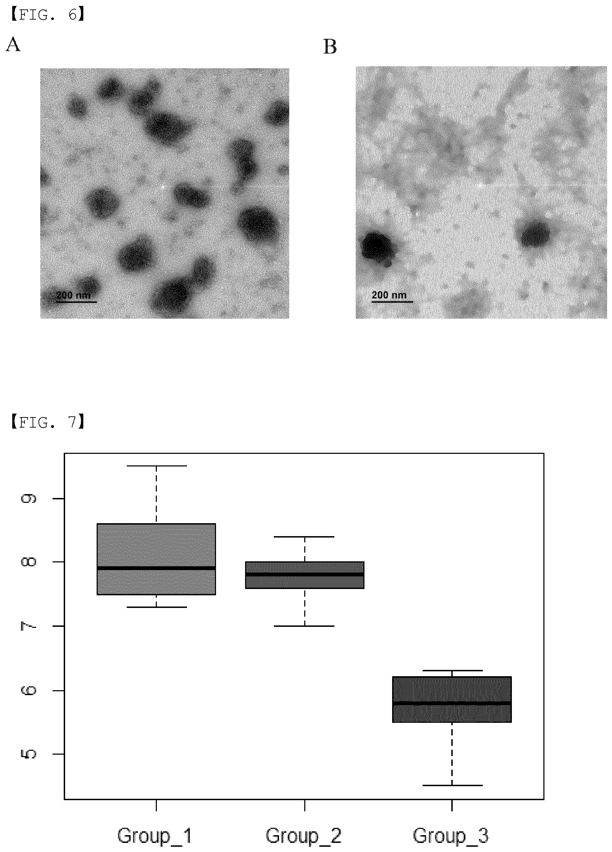

FIG. 6 shows electron microscope images of remdesivir nanoparticles prepared according to Example 1 of the present disclosure and glutathione nanoparticles prepared according to Example 2 of the present disclosure in Experimental Example 1 of the present disclosure (FIG 6A: remdesivir nanoparticles and FIG. 6B: glutathione nanoparticles);
FIG. 7 shows analysis results of gut microbiome diversity using the Shannon alpha-diversity analysis program after metagenome sequencing of the gut microbiome of mice that are orally administered azithromycin nanoparticles in Experimental Example 2 of the present disclosure (Group 1: control group not involving antibiotics, Group 2: group administered azithromycin nanoparticles, and Group 3: group administered azithromycin);

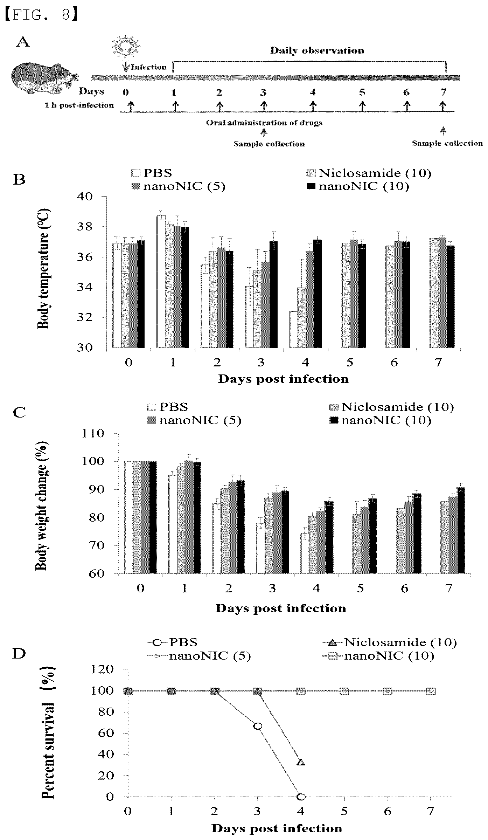

FIG. 8 shows confirmation results of antiviral treatment efficacy after oral administration of niclosamide or niclosamide nanoparticles to SH101 hamsters, an animal model infected with a coronavirus, in Experimental Example 3 of the present disclosure (FIG. 8A: experimental design and schedule diagram of antiviral efficacy evaluation, FIG. 8B: measurement results of body temperature, FIG. 8C: measurement results of body weight, and FIG. 8D: measurement results of survival rate (% survival));

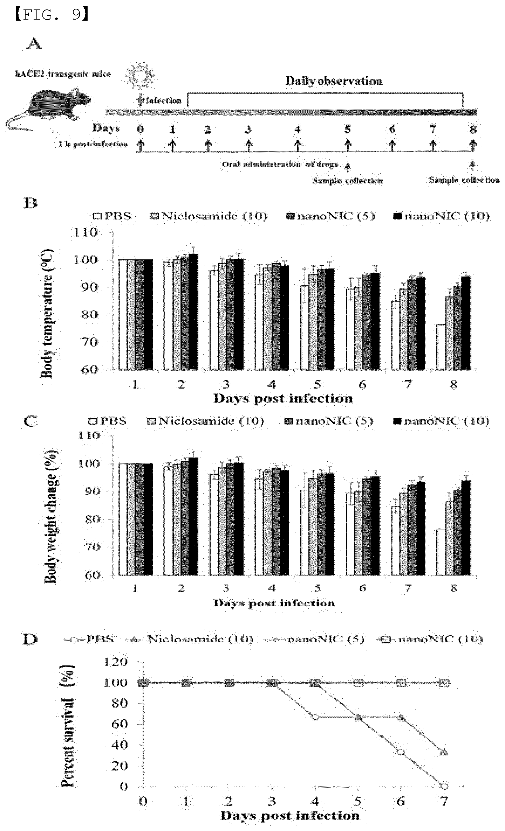

FIG. 9 shows confirmation results of antiviral treatment efficacy after oral administration of niclosamide or niclosamide nanoparticles to hACE transgenic mice, an animal model infected with a coronavirus, in Experimental Example 3 of the present disclosure (FIG. 9A: experimental design and schedule diagram of antiviral efficacy evaluation, FIG. 9B: measurement results of body temperature, FIG. 9C: measurement results of body weight, and FIG. 9D: measurement results of survival rate (% survival)); and

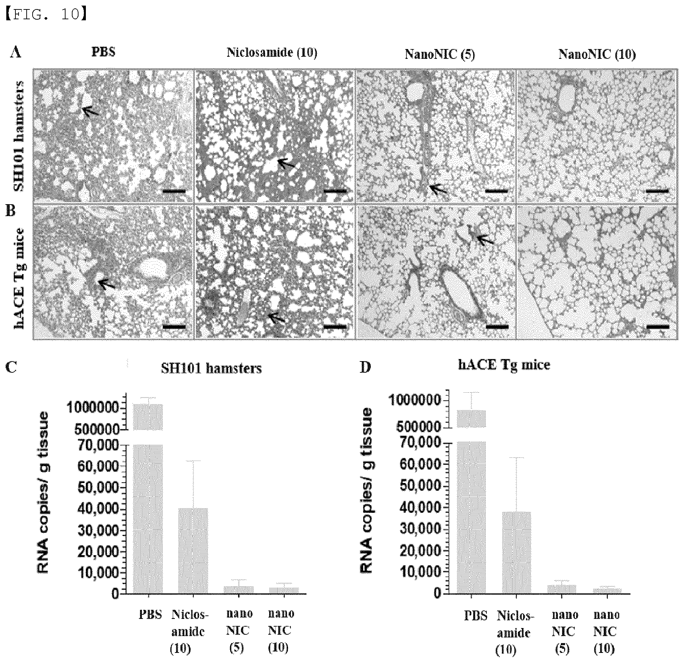

FIG. 10 shows observation results of lung tissue obtained after oral administration of niclosamide or niclosamide nanoparticles to SH101 hamsters and hACE transgenic mice, animal models infected with a coronavirus, in Experimental Example 3 of the present disclosure (arrow mark: inflammation sites caused by infection) and quantitative analysis results of coronavirus levels in lung tissue (FIG. 10A: H&E-stained images of SH101 hamster lung tissue, FIG. 10B: H&E-stained image of hACE transgenic mouse lung tissue, FIG. 10C: SARS-CoV-2 viral RNA copies per 1 gram of SH101 hamster lung tissue, and FIG. 10D: SARS-CoV-2 viral RNA copies per 1 gram of hACE transgenic mouse lung tissue).

### Best Mode

Among medications that have efficacy in all types of diseases, oral medications are easy to take and economically feasible to the extent that injections are incomparable. Thus, various efforts have been made to improve low oral bioavailability of medications. However, in the technologies invented so far, improvement in bioavailability has been insignificant, which makes the formulation of oral medications to replace injections difficult or the degradation of drugs by digestive enzymes unavoidable. Alternatively, there has been a serious problem in that all the structure, physical properties, and chemical properties of drugs are changed due to the use of a method of being chemically bonded to drugs. For this reason, there are hardly any cases where such technologies succeed and are commonly used, so many drugs are being used as injections or oral medications having extremely low bioavailability despite several drawbacks. Therefore, in order to t replace injections with oral medications, there is a need for inventions with innovative ideas. All of the problems in oral medications described above are capable of being solved in the present disclosure, enabling all injections to be replaced with oral tablets. Thus, once universalized, the present disclosure is expected to bring revolution in medical treatment to mankind.

In existing inventions, a chemical bond, such as a covalent bond, allows a bioactive chemical compound to be linked to bile acids. Unlike in existing inventions, the inventors of the present disclosure focused on performing a process of encapsulating a desired compound with bile acid to non-covalently prepare a stable nanoparticle without involving any covalent bond. Once this is enabled, the bile acid present on the surface of the nanoparticle and the chemical compound present in a core portion are unlikely to be structurally changed and thus are enabled to be absorbed into the blood with ultra-high efficiency through enterohepatic circulation, an absorption pathway of the bile acid, while maintaining the chemical properties thereof as they are. As a result, the present disclosure was completed.

The preparation of currently available oral nanoparticles essentially requires a process of covalent bonding (conjugation), which causes chemical changes in reactants in all cases. For this reason, the nanoparticle preparation process is extremely complicated. In addition, due to the covalent bond, reactants of the chemical compound are structurally changed, thereby losing the unique properties thereof. Thus, the original function of the drug may be hardly maintained, and at the same time, unexpected side effects may occur due to novel chemical entities. Furthermore, the nanoformulation and the drug are required to be able to react chemically, so the types of drugs capable of being encapsulated in the nanoparticle are limited.

In the oral nanoparticle of the present disclosure, the two exact components, ① a compound to be absorbed into the body and ② bile acid serving as a drug carrier surrounding the compound, are linked only by a non-covalent bond therebetween. Thus, the oral nanoparticle of the present disclosure has the following effects of 1) dramatically improving the bioavailability of the encapsulated compound, 2) preventing modification or degradation in digestive organs by minimizing the exposure of the encapsulated compound to digestive juices, 3) increasing intestinal permeability by reabsorbing both of the encapsulated compound and the bile acid into the blood, and 4) maintaining and protecting the balance in the gut microbiome by minimizing the exposure of the encapsulated compound to intestinal microorganisms. Due to such excellent advantages, the nanoparticle of the present disclosure is an innovative invention capable of changing all drugs currently used as injections to oral preparations.

In one embodiment of the present disclosure, provided are an oral nanoparticle in which the bioavailability of a compound is low, formulation of the same, and a use of the same.

Therefore, one aspect of the present disclosure relates to the oral nanoparticle composed of a bioactive compound present in a core portion and bile acid surrounding the bioactive compound, which is characterized in that the bioactive compound and the bile acid are non-covalently bonded.

The bioactive compound includes drugs, nutritional ingredients, and all functional materials intended to be absorbed into the body in medications, nutritional supplements, and dietary supplements. Specific examples thereof may include 5-fluorouracil, remdesivir, azithromycin, paclitaxel, doxorubicin, oxaliplatin, phenylephrine hydrochloride, glutathione, acetazolamide, amphotericin, aprepitant, azathioprine, chlorothiazide, chlorthalidone, ciprofloxacin, colistin, cyclosporin A, digoxin, docetaxel, furosemide, etravirine, famotidine, griseofulvin, hydrochlorothiazide, mebendazole, methotrexate, neomycin, niclosamide, nystatin, ritonavir, albendazole, artemther, chlorpromazine, efavirenz, glibenclamide, ivermectin, lopinavir, mefloquine, retinol, spironolactone, sulfadiazine, sulfasalazine, triclabendazole, acyclovir, amoxicillin, bidisomide, biperiden, captopril, cefazolin, chloroquine, cimetidine, cloxacillin, didanosine, ephedrine, erythromycin, famotidine, fluconazole, folinic acid, furosemide, ganciclovir, lisinopril, methotrexate, metformin, nifurtimox, nadolol, nystatin, pravastatin, penicillin, ranitidine, reserpine, tetracycline, valsartan, vancomycin, doxycycline, chlorphenamine, clomiphene, clomipramine, dexamethasone, ethinylestradiol, metoclopramide, morphine, quinine, and the like, but are not limited thereto.

The bile acid is characterized by being amphiphilic while being actively absorbed into the gut and preferably includes bile acids or derivatives thereof. Specific examples thereof may include cholic acid, chenodeoxycholic acid, deoxycholic acid, lithocholic acid, ursodeoxycholic acid, tauroursodeoxycholic acid, hyodeoxycholic acid, 7-oxo lithocholic acid, iododeoxycholic acid, iodine cholic acid, taurolithocholic acid, glycoursodeoxycholic acid, taurocholic acid, glycocholic acid, and the like, but are not limited thereto.

In addition, the bioactive compound and the bile acid are only non-covalently bonded to each other without involving any covalent bond, so the "oral nanoparticle for the bioactive compound" of the present disclosure is characterized in that the chemical properties thereof are maintained without causing any chemical modification.

Another aspect of the present disclosure relates to an oral nanoparticle composition containing the oral nanoparticle.

The oral nanoparticle composition of the present disclosure may include a pharmaceutically acceptable carrier commonly used in the formulation. Examples of the carrier may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylhydroxybenzoate, propylhydroxybenzoate, talc, magnesium stearate, mineral oil and the like, but are not limited thereto.

The oral nanoparticle composition of the present disclosure may further contain a lubricant, a wetting agent, a sweetening agent, a flavoring agent, an emulsifier, a suspending agent, a preservative, and the like in addition to the components described above. Suitable preparations and carriers pharmaceutically acceptable are described in detail in Remington's Pharmaceutical Sciences (19th ed., 1995).

The oral nanoparticle composition of the present disclosure may be prescribed in an appropriate dose that varies depending on factors such as a formulation method, an administration method, age, weight, and sex of patients, medical conditions, food, administration period, administration route, excretion rate, and reaction sensitivity. On the other hand, the oral nanoparticle composition of the present disclosure is preferably administered at a dose in a range of 0.0001 to 1000 µg per day.

The oral nanoparticle composition of the present disclosure may be prepared in a unit dosage form through formulation using a pharmaceutically acceptable carrier and/or excipient, or may be prepared in a multi-dose container, depending on methods easily implementable by those skilled in the art to which the present disclosure belongs. In this case, the composition may be formulated in the form of a solution in an oil or aqueous medium, suspension, or emulsion, or the form of an extract, powder, granule, tablet, or capsule. In addition, a dispersant or stabilizer may be additionally contained.

The oral nanoparticle composition of the present disclosure is capable of keeping modification or degradation from occurring before being absorbed in the digestive tract by minimizing the exposure of the compound encapsulated in the nanoparticle to digestive enzymes.

In addition, the oral nanoparticle composition of the present disclosure is capable of maintaining and protecting the balance in the gut microbiome by minimizing the exposure of the compound encapsulated in the nanoparticle to intestinal microorganisms.

A further aspect of the present disclosure relates to a method of preparing an oral nanoparticle, which includes: (a) dissolving a bioactive compound in a solvent to prepare a bioactive compound solution, (b) dissolving bile acid in a solvent to prepare a bile acid solution, (c) mixing the bioactive compound solution and the bile acid solution, and (d) lyophilizing the resulting mixed solution of the bioactive material and bile acid.

Examples of the solvent in which the bioactive compound is dissolved may include water, DMSO, ethanol, methanol, acetone, and the like, but are not limited thereto.

Examples of the solvent in which the bile acid is dissolved may include ethanol, water, methanol, and the like, but are not limited thereto.

To prepare the oral nanoparticle of the present disclosure, composed of the bioactive compound present in a core portion and the bile acid surrounding the bioactive compound, in which the bioactive compound and the bile acid are non-covalently bonded, the bioactive compound solution and the bile acid solution are required to be mixed. Such mixing may be performed by mixing the bioactive compound solution and the bile acid solution in one place, and then stirring or ultrasonicating the resulting mixed solution.

To further facilitate the formation of the oral nanoparticle, the method of preparing the oral nanoparticle of the present disclosure may further include subjecting the resulting mixed solution of the bioactive compound and the bile acid to low-temperature treatment, salt treatment, or salt treatment followed by low-temperature treatment, after mixing the bioactive compound solution and the bile acid solution.

The low-temperature treatment may be performed by lowering the temperature from room temperature to a temperature in a range of -20°C to +20°C at a rate of 1°C/min or lower while stirring the mixed solution of the bioactive compound and the bile acid. In addition, the salt treatment may be performed by adding a salt of a cation selected from the group consisting of Na⁺, Mg²⁺, Li⁺, Ca²⁺, Fe²⁺, and the like in an amount corresponding up to a concentration of 0.1 to 20 M while stirring the resulting mixed solution of the bioactive compound and the bile acid.

When the rate or temperature does not fall within the above numerical range during the low-temperature treatment, the effect of facilitating the formation of the oral nanoparticle may be insignificant.

In addition, when the concentration of the salt treated does not fall within the above numerical range during the salt treatment, the effect of facilitating the formation of the oral nanoparticle may be insignificant.

### Mode for Invention

Hereinafter, the present disclosure will be described in more detail through examples. These examples are only for illustrating the present disclosure, and it will be apparent to those skilled in the art that the scope of the present disclosure is not to be construed as being limited by these examples.

### Example 1. Preparation of oral nanoparticles by mixture reaction

Niclosamide is known to have a number of excellent pharmacological effects, such as antibacterial, antiparasitic, anticancer, antiviral, and neuroprotective effects. However, due to the problems of absorption and water insolubility, FDA approves niclosamide only as a drug for intestinal parasites, which is unnecessary to be absorbed into the body. Despite the excellent antibacterial and antiviral effects thereof, niclosamide fails to be developed as a coronavirus (COVID-19) treatment, tuberculosis treatment, antiviral treatment, or anticancer treatment. This is because niclosamide, a hydrophobic chemical compound, is only dissolved in limited hydrophobic organic solvents, making the development thereof as an injection difficult. In addition, niclosamide is hardly absorbed into the body when orally administered, so the bioavailability of the tablet is lower than 10%. Furthermore, paclitaxel, a representative cytotoxic anticancer drug, is also prescribed only as an injection because the bioavailability thereof is about 10%. When formulated into oral nanoparticles, paclitaxel may be prescribed as an oral medication instead of an injection, thereby reducing side effects and improving the ease of use.

Hence, the inventors of the present disclosure formed nanoparticles of niclosamide and paclitaxel, expected to show the effect of increasing dose, reduce side effects, and be easy to use when being developed as an oral preparation having improved bioavailability, as follows.

Niclosamide powder was dissolved in a DMSO solvent at a concentration of 6 mg/mL, and cholic acid powder was dissolved in water at a concentration of 6 mg/mL. After mixing 1 mL of the niclosamide solution and 3 mL of the cholic acid solution, the two solutions were thoroughly mixed in an ultrasonic generator filled with water for 5 minutes, and then the resulting mixed solution was lyophilized for pulverization. Unlike pure niclosamide, the prepared niclosamide nanoparticles, completely water-soluble nanoparticles, were well-soluble in water without any precipitate, and had an average particle size in a range of 120 to 150 nm, which was confirmed by an electron microscope (FIG. 1).

In addition, it was confirmed that the particle size was maintained stably even when the prepared niclosamide nanoparticles were stored for a long term of more than 6 weeks or left for more than 12 hours at pH 6.8 and pH 7.4 (FIG. 2)

Paclitaxel powder was dissolved in DMSO at a concentration of 10 mg/mL, and cholic acid powder was dissolved in ethanol at a concentration of 6 mg/mL. After mixing 1 mL of the paclitaxel solution and 3 mL of the cholic acid solution, a container containing the resulting mixed solution was placed in an ultrasonic generator filled with water, and then the resulting mixed solution was thoroughly mixed by ultrasound for 5 minutes. After completion of the mixing process, the resulting mixed was lyophilized for pulverization.

Unlike pure paclitaxel, the prepared paclitaxel nanoparticles, completely water-soluble nanoparticles, were well-soluble in water, and had an average particle size in a range of 150 to 250 nm, which was confirmed by an electron microscope and a particle size analyzer (FIG. 3).

Rats were orally administered each of the prepared nanoparticles at a dose of 20 mg/Kg (based on the experimental compound). Then, blood was collected from the rats hourly. In this case, as a control group, the same experiment was performed by orally administering the pure bioactive compounds used when preparing the respective nanoparticles in the same amount. After adding 0.9 mL of acetone to 0.1 mL of the blood collected from the rats hourly, the resulting mixed solution of the two was thoroughly mixed using a vortex. The resulting mixed solution was centrifuged at a speed of 15,000 rpm to remove the precipitate, and only the obtained solution was analyzed by LC-MS/MS. As a result, the bioavailability is as shown in Table 1.

**[Table 1]**

| Types of bioactive compound | Bioavailability of chemical compound not involving nanoparticle formation | Bioavailability of chemical compound formed into nanoparticles (nanoparticles containing cholic acid for encapsulation) |
|---|---|---|
| Niclosamide | 9.5 % | 75.3 % |
| Paclitaxel | 10.4 % | 89.7 % |

This experiment described the process of preparing the nanoparticles of niclosamide and paclitaxel. However, hydrophobic compounds having low body absorption among the BCS classification system class IV group (acetazolamide, amphotericin, aprepitant, azathioprine, chlorothiazide, chlorthalidone, ciprofloxacin, colistin, cyclosporin A, digoxin, docetaxel, furosemide, etravirine, famotidine, furosemide, griseofulvin, hydrochlorothiazide, mebendazole, methotrexate, neomycin, nystatin, paclitaxel, ritonavir, and the like) or hydrophobic compounds, other than the class IV group, that need to improve body absorption (albendazole, artemther, chlorpromazine, efavirenz, glibenclamide, ivermectin, lopinavir, mefloquine, retinol, spironolactone, sulfadiazine, sulfasalazine, triclabendazole, and the like) may also be prepared into the oral nanoparticles of the present disclosure in a similar manner. In such cases, the oral nanoparticles were confirmed to be prepared even when using ethanol, methanol, acetone, and the like as the solvent instead of DMSO or using other types of bile acids instead of cholic acid.

### Example 2. Preparation of oral nanoparticles through salt treatment

5-Fluorouracil, a representative cytotoxic anticancer drug, is one of the most commonly used anticancer drugs for cancer treatment, but the oral bioavailability thereof is low, and is thus currently being used only as an injection. Metformin, a representative treatment for type 2 diabetes, is easy to take and thus mainly prescribed as a tablet, but the bioavailability thereof is only at a level of 30%.

Hence, the inventors of the present disclosure performed experiments on 5-fluorouracil and metformin, expected to show the effect of increasing dose, reduce side effects, and be easy to use when being developed as an oral preparation having improved bioavailability. As a result of experiments performed by varying conditions using the technology of the present disclosure, nanoparticles were formed as follows.

5-Fluorouracil powder was dissolved in water at a concentration of 6 mg/mL, and cholic acid powder was dissolved in ethanol at a concentration of 6 mg/mL. After mixing 1 mL of the 5-fluorouracil solution and 3 mL of the cholic acid solution, 10 mg of NaCl was mixed in the resulting mixed solution, and the two solutions were thoroughly mixed in an ultrasonic generator filled with ethanol for 5 minutes. Next, with the addition of dry ice to the ultrasonic generator while maintaining the occurrence of ultrasound, the 5-fluorouracil solution was frozen and then lyophilized for pulverization. The prepared 5-fluorouracil nanoparticles were confirmed to have a size in a range of 90 to 120 nm (FIG. 4).

Metformin powder was dissolved in water at a concentration of 5 mg/mL, and cholic acid powder was dissolved in ethanol at a concentration of 5 mg/mL. After mixing 1 mL of the metformin solution and 3 mL of the cholic acid solution, 60 mg of NaCl was added to the resulting mixed solution, and the two solutions were thoroughly mixed in an ultrasonic generator filled with ethanol for 5 minutes. Next, with the addition of dry ice to the ultrasonic generator while maintaining the occurrence of ultrasound, the metformin solution was frozen and then lyophilized for pulverization. The prepared metformin nanoparticles were confirmed to have a size in a range of 90 to 120 nm (FIG. 4).

Rats were orally administered each of the prepared nanoparticles at a dose of 20 mg/Kg (based on the bioactive compound). Then, blood was collected from the rats hourly. As a control group, the same experiment was performed by orally administering the pure bioactive compounds used when preparing the respective nanoparticles in the same amount. After adding 0.9 mL of acetone to 0.1 mL of the blood collected from the rats hourly, the resulting mixed solution of the two was thoroughly mixed using a vortex. The resulting mixed solution was centrifuged at a speed of 15,000 rpm to remove the precipitate, and only the obtained solution was analyzed by LC-MS/MS. As a result, the bioavailability is as shown in Table 2.

**[Table 2]**

| Types of bioactive compound | Bioavailability of chemical compound not involving nanoparticle formation | Bioavailability of chemical compound formed into nanoparticles (nanoparticles containing cholic acid for encapsulation) |
|---|---|---|
| 5-Fluorouracil | 57.8 % | 95.3 % |
| Metformin | 38.3 % | 81.7 % |

This experiment described the process of preparing the nanoparticles of 5-fluorouracil and metformin. However, hydrophilic compounds having low permeability among the BCS classification system class III group (acyclovir, amoxicillin, bidisomide, biperiden, captopril, cefazolin, chloroquine, cimetidine, cloxacillin, didanosine, ephedrine, erythromycin, famotidine, fluconazole, folinic acid, furosemide, ganciclovir, lisinopril, methotrexate, metformin, nifurtimox, nadolol, nystatin, pravastatin, penicillin, ranitidine, reserpine, tetracycline, valsartan, vancomycin, and the like), or hydrophilic compounds, other than the class III group, that need to improve body absorption (chlorpheniramine, clomiphene, clomipramine, dexamethasone, ethinylestradiol, metoclopramide, morphine, quinine, and the like) may also be prepared into the oral nanoparticles of the present disclosure in a similar manner. In such cases, the oral nanoparticles were confirmed to be prepared even when using ethanol, methanol, acetone, and the like as the solvent instead of water or using other types of bile acids instead of cholic acid, as needed.

### Example 3. Preparation of oral nanoparticles through low-temperature treatment

Azithromycin, a representative broad-spectrum antibiotic, is one of the most commonly used antibiotics for bacterial infection treatment, but the oral bioavailability thereof is low, and thus remains in large amounts in the gut without being absorbed into the human body. Azithromycin remaining in the gut kills intestinal microorganisms and destroys the intestinal flora, causing a number of serious side effects in people.

Ciprofloxacin is also a representative broad-spectrum antibiotic, but the bioavailability thereof is not high, causing the same problems as in azithromycin. Therefore, there is a need to reduce the side effects of drugs by improving bioavailability and reducing residual amounts in the intestine.

Hence, the inventors of the present disclosure performed experiments on azithromycin and ciprofloxacin, expected to show the effect of increasing dose, reduce side effects, and be easy to use when being developed as an oral preparation having improved bioavailability. As a result of the experiments performed by varying conditions using the technology of the present disclosure, nanoparticles were formed as follows.

Azithromycin powder was dissolved in ethanol at a concentration of 6 mg/mL, and cholic acid powder was dissolved in ethanol at a concentration of 6 mg/mL. Next, after mixing 1 mL of the azithromycin solution and 3 mL of the cholic acid solution, 1 mg of NaCl was mixed in the resulting mixed solution, and the two solutions were thoroughly mixed in an ultrasonic generator filled with ethanol for 5 minutes. Then, using a temperature controller, the temperature was lowered from room temperature to -4°C at a rate of 0.001°C/min while stirring the resulting mixed solution of azithromycin and cholic acid. Lastly, the resulting mixed solution was frozen and lyophilized for pulverization. The prepared azithromycin nanoparticles were confirmed to have a size in a range of 130 to 220 nm (FIG. 5).

Ciprofloxacin powder was dissolved in water at a concentration of 5 mg/mL, and cholic acid powder was dissolved in ethanol at a concentration of 5 mg/mL. After mixing 1 mL of the ciprofloxacin solution and 3 mL of the cholic acid solution, 1 mg of FeCl₂ was added to the resulting mixed solution, and the two solutions were thoroughly mixed in an ultrasonic generator filled with ethanol for 5 minutes. Then, using a temperature controller, the temperature was lowered from room temperature to -2°C at a rate of 0.001°C/min while stirring the resulting mixed solution of ciprofloxacin and cholic acid. Lastly, the resulting mixed solution was frozen and lyophilized for pulverization. The prepared ciprofloxacin nanoparticles were confirmed to have a size in a range of 100 to 200 nm (FIG. 5).

Rats were orally administered each of the prepared nanoparticles at a dose of 20 mg/Kg (based on the bioactive compound). Then, blood was collected from the rats hourly. As a control group, the same experiment was performed by orally administering the pure bioactive compound used when preparing the respective nanoparticles in the same amount. After adding 0.9 mL of acetone to 0.1 mL of the blood collected from the rats hourly, the resulting mixed solution of the two was thoroughly mixed using a vortex. The resulting mixed solution was centrifuged at a speed of 15,000 rpm to remove the precipitate, and only the obtained solution was analyzed by LC-MS/MS. As a result, the bioavailability is as shown in Table 3.

**[Table 3]**

| Types of bioactive compound | Bioavailability of chemical compound not involving nanoparticle formation | Bioavailability of chemical compound formed into nanoparticles (nanoparticles containing cholic acid for encapsulation) |
|---|---|---|
| Azithromycin | 32.1 % | 83.2 % |
| Ciprofloxacin | 19.3 % | 89.5 % |

This experiment described the process of preparing the nanoparticles of azithromycin and ciprofloxacin. However, compounds having low permeability among the BCS classification system class III group (chloroquine, cimetidine, cloxacillin, didanosine, ephedrine, erythromycin, famotidine, fluconazole, folinic acid, furosemide, ganciclovir, lisinopril, methotrexate, metformin, nifurtimox, nadolol, nystatin, pravastatin, penicillin, ranitidine, reserpine, tetracycline, valsartan, vancomycin, doxycycline, and the like), or hydrophilic compounds, other than the class III group, that need to improve body absorption (chlorpheniramine, clomiphene, clomipramine, dexamethasone, ethinylestradiol, metoclopramide, morphine, quinine, and the like) are also prepared into the oral nanoparticles of the present disclosure in a similar manner. In such cases, the oral nanoparticles were confirmed to be prepared even when using ethanol, methanol, acetone, and the like as the solvent instead of water or using other types of bile acids instead of cholic acid, as needed.

### Experimental Example 1. Evaluation of nanoparticle formation effect of present disclosure on degradation of encapsulated compound

Remdesivir is one type of nucleoside analog and is the first approved coronavirus treatment due to the excellent antiviral effect thereof. Remdesivir is the most effective treatment for COVID-19 but has only been developed as an injection. This is because when ingested, remdesivir is immediately degraded by the nuclease in digestive juices in many cases, so the bioavailability thereof when used as an oral medication is extremely low compared to that of the remdesivir when used as the injection that is absorbed into the blood.

Glutathione, derived from a peptide formed by a peptide bond of three amino acids, has a strong antioxidant effect as well as a skin-lightening effect. When taken, glutathione is immediately degraded by proteolytic enzymes in digestive juices, and only an extremely small amount is absorbed, so the bioavailability of glutathione is extremely low. Therefore, when orally taking glutathione, the desired skin-lightening effect is difficult to be expected. For this reason, glutathione is used as an injection rather than as an oral medication. Due to a number of disadvantages in injections, research on improving the bioavailability of glutathione is actively in progress. However, the problem of glutathione being degraded by digestive enzymes is yet to be solved, and there have been no successful cases of developing oral preparations of glutathione.

Hence, in order to develop oral preparations having improved bioavailability, the inventors of the present disclosure performed experiments by varying conditions using the technology of the present disclosure. As a result of the experiments, remdesivir nanoparticles for oral administration were prepared using the technology of Example 1 (FIG. 6), and glutathione nanoparticles for oral administration were prepared using the technology of Example 2 (FIG. 6).

Rats were orally administered each of the prepared remdesivir nanoparticles and glutathione nanoparticles at a dose of 20 mg/Kg (based on the drug). Then, blood was collected from the rats hourly. As a control group, the same experiment was performed by orally administering the pure bioactive compound (remdesivir or glutathione) used when preparing the nanoparticle in the same amount. After adding 0.9 mL of acetone to 0.1 mL of the blood collected from the rats hourly, the resulting mixed solution of the two was thoroughly mixed using a vortex. The resulting mixed solution was centrifuged at a speed of 15,000 rpm to remove the precipitate, and only the obtained solution was analyzed by LC-MS/MS. As a result, the bioavailability is as shown in Table 4.

**[Table 4]**

| Types of bioactive compound | Bioavailability of chemical compound not involving nanoparticle formation | Bioavailability of chemical compound formed into nanoparticles (nanoparticles containing cholic acid for encapsulation) |
|---|---|---|
| Remdesivir | 1.5 % | 89.1% |
| Glutathione | 9.1 % | 83.3 % |

As seen in Table 4, the present disclosure, an innovative technology that enables bioactive compounds, which have been only available as injections due to the problem of being digested when orally administered, to be formulated into oral medications, greatly improves the bioavailability by protecting the bioactive compounds from digestive enzymes in the body.

Although this experiment shows the process of preparing the nanoparticles of remdesivir and glutathione as examples, other biochemical compounds characterized by being degraded by digestive enzymes in the digestive organs when orally administered are also prepared into the oral nanoparticle of the present disclosure in a similar manner. In such cases, the oral nanoparticles were confirmed to be prepared even when using ethanol, methanol, acetone, and the like as the solvent instead of DMSO or using other types of bile acids instead of cholic acid, as needed.

### Experimental Example 2. Evaluation of nanoparticle formation effect of present disclosure on equilibrium in gut microbiome

Amoxicillin, azithromycin, clindamycin, ceftriaxone, erythromycin, fluconazole, penicillin, tetracycline, and the like are antibiotics commonly used in hospitals. Most of these antibiotics have excellent bioavailability due to the excellent solubility and permeability thereof and thus are typically used as oral medications instead of injections. However, antibiotics present in the intestine after being orally administered and remaining in the intestinal lumen until absorbed into the blood kill the surrounding intestinal microorganisms, causing the gut microbiome to be out of equilibrium and thus leading to a number of digestive system diseases such as diarrhea, abdominal pain, and the like.

Hence, in order to analyze the effect on the equilibrium in the gut microbiome during nanoparticle formation of antibiotics, the inventors of the present disclosure prepared oral nanoparticles of azithromycin using the technology of Example 3. Rats were orally administered the prepared nanoparticles at a dose of 20 mg/Kg. Then, blood was collected from the rats hourly. In this case, a comparison experiment was performed by orally administering the same amount of azithromycin to rats of a control group. After adding 0.9 mL of acetone to 0.1 mL of the blood collected from the rats hourly, the resulting mixed solution of the two was thoroughly mixed using a vortex. The resulting mixed solution was centrifuged at a speed of 15,000 rpm to remove the precipitate, and only the obtained solution was analyzed by LC-MS/MS. As a result, the bioavailability is as shown in Table 5.

**[Table 5]**

| Types of bioactive compound | Bioavailability of chemical compound not involving | Bioavailability of chemical compound formed into nanoparticles (nanoparticles |
|---|---|---|
| | nanoparticle formation | containing cholic acid for encapsulation) |
| Azithromycin | 39.8 % | 92.6.% |

Each Rat was orally administered the azithromycin nanoparticles prepared by the method of Example 3 at a dose of 5 mg/Kg (based on the drug) daily for 5 days, and then the gut microbiome was analyzed by metagenome sequencing. In this case, as a control group, the same experiment was performed by orally administering the original bioactive compound (azithromycin) to animals in the same amount as the nanoparticles. As a result of the experiment, it was seen that in the case of the group administered the azithromycin nanoparticles, the gut microbiome was kept from being out of equilibrium, compared to the case of the group administered azithromycin, because azithromycin, the antibiotic, was kept from being exposed to the gut microbiome (FIG. 7).

### Experimental Example 3. Evaluation of nanoparticle formation effect of present disclosure on drug efficacy

The inventors of the present disclosure confirmed the structural stability and improved bioavailability of the niclosamide nanoparticles prepared by the method of Example 1, and then confirmed the treatment effect of the oral niclosamide nanoparticles on viral infections through in vivo animal experiments. All experiments were performed at BSL level 3. To this end, SH101 hamsters, a coronavirus animal model, were first prepared and then infected with a lethal dose (TCID₅₀) of the SARS-CoV-2 virus. When one hour had elapsed from the viral infection, the niclosamide nanoparticles were orally administered daily at a dose of 5 or 10 mg/Kg (based on the drug). In this case, as a control group, a comparison experiment was performed by orally administering 10 mg/Kg of the original bioactive compound, niclosamide, or phosphate-buffered saline (PBS). Next, infection symptoms (such as weight loss, hypothermia after fever, somatic death, and the like) were confirmed for 7 day-post-infection (dpi) (FIG. 8).

The same experiment was performed using hACE2 transgenic mice, another coronavirus animal model, while confirming infection symptoms (such as weight loss, hypothermia after fever, somatic death, and the like) for 7 day-post-infection (dpi) (FIG. 9).

Fragments of each lung tissue sampled at 3 dpi and 7 dpi were stained with hematoxylin and eosin (H&E) and then observed using a microscope (FIG. 10). In addition, regarding RNA extracted from the lung tissue, PCR (forward primers: 5'-GCCTCTTCTCGTTCCTCATCAC-3', reverse primer: 5'-AGCAGCATCACCGCCATTG-3') was performed on the envelope (E) gene of the SARS-CoV-2 virus at 50°C for 2 minutes, at 95°C for 2 minutes, and then [95°C for 15 seconds and 60°C for 30 seconds] at 40 cycles. Then, RT-qPCR was performed under the following conditions: 95°C for 15 seconds, 60°C for 1 minute, and 95°C for 45 seconds. As a result, the amount of the SARS-CoV-2 virus in the lung tissue was quantitatively analyzed (FIG. 10).

As described above, according to the results of the experiments performed on SH101 hamsters and hACE2 transgenic mice, the coronavirus animal models, in the group administered the niclosamide nanoparticles, niclosamide, an antiviral agent, was delivered to the infected tissue. As a result, the bioavailability was improved, so the SARS-CoV-2 virus was killed (FIGS. 10C and 10D). Therefore, unlike in the group administered either PBS or niclosamide, a noticeable treatment effect on SARS-CoV-2 infection was confirmed to be exhibited.

### Industrial Applicability

A nanoparticle of the present disclosure is an innovative invention that enables all drugs currently used as injections to be changed to oral preparations.

## Claims

1. An oral nanoparticle comprising:
a bioactive compound present in a core portion; and
bile acid surrounding the bioactive compound,
wherein the bioactive compound and the bile acid are non-covalently bonded.

2. The nanoparticle of claim 1, wherein the bioactive compound is contained in an amount in a range of 10% to 90% by weight, and the bile acid is contained in an amount in a range of 10% to 90% by weight.

3. The nanoparticle of claim 1, wherein the bioactive compound comprises one or more selected from the group consisting of 5-fluorouracil, remdesivir, azithromycin, paclitaxel, doxorubicin, oxaliplatin, phenylephrine hydrochloride, glutathione, acetazolamide, amphotericin, aprepitant, azathioprine, chlorothiazide, chlorthalidone, ciprofloxacin, colistin, cyclosporin A, digoxin, docetaxel, furosemide, etravirine, famotidine, griseofulvin, hydrochlorothiazide, mebendazole, methotrexate, neomycin, niclosamide, nystatin, ritonavir, albendazole, artemther, chlorpromazine, efavirenz, glibenclamide, ivermectin, lopinavir, mefloquine, retinol, spironolactone, sulfadiazine, sulfasalazine, triclabendazole, acyclovir, amoxicillin, bidisomide, biperiden, captopril, cefazolin, chloroquine, cimetidine, cloxacillin, didanosine, ephedrine, erythromycin, famotidine, fluconazole, folinic acid, furosemide, ganciclovir, lisinopril, methotrexate, metformin, nifurtimox, nadolol, nystatin, pravastatin, penicillin, ranitidine, reserpine, tetracycline, valsartan, vancomycin, doxycycline, chlorpheniramine, clomiphene, clomipramine, dexamethasone, ethinylestradiol, metoclopramide, morphine, and quinine.

4. The nanoparticle of claim 1, wherein the bile acid comprises one or more selected from the group consisting of cholic acid, chenodeoxycholic acid, deoxycholic acid, lithocholic acid, ursodeoxycholic acid, tauroursodeoxycholic acid hyodeoxycholic acid, 7-oxo lithocholic acid, iododeoxycholic acid, iodine cholic acid, taurolithocholic acid, glycoursodeoxycholic acid, taurocholic acid, and glycocholic acid.

5. A method of preparing an oral nanoparticle, the method comprising:
(a) dissolving a bioactive compound in a solvent to prepare a bioactive compound solution;
(b) dissolving bile acid in a solvent to prepare a bile acid solution;
(c) mixing the bioactive compound solution and the bile acid solution; and
(d) lyophilizing the resulting mixed solution of the bioactive compound and the bile acid.

6. The method of claim 5, wherein a volume ratio of the bioactive compound solution to the bile acid solution is in a range of 10 to 90:90 to 10.

7. The method of claim 5, further comprising subjecting the resulting mixed solution of the bioactive compound and the bile acid to low-temperature treatment or salt treatment after the mixing of the bioactive compound solution and the bile acid solution.

8. The method of claim 7, wherein the low-temperature treatment is performed by lowering a temperature from room temperature to a temperature in a range of -20°C to +20°C at a rate of 1°C/min or lower while stirring the resulting mixed solution of the bioactive compound and the bile acid.

9. The method of claim 7, wherein the salt treatment is performed by adding a salt of a cation selected from the group consisting of Na⁺, Mg²⁺, Li⁺, Ca²⁺, and Fe²⁺ in an amount corresponding up to a concentration of 0.1 to 20 M while stirring the resulting mixed solution of the bioactive compound and the bile acid.

10. An oral nanoparticle composition containing the oral nanoparticle of any one of claims 1 to 4.
